(19) Europäisches Patentamt
European Patent Office
Office européen des brevets

(11) Veröffentlichungsnummer: **0 014 887**
**B1**

# (12) EUROPÄISCHE PATENTSCHRIFT

(45) Veröffentlichungstag der Patentschrift: 14.10.81

(51) Int Cl.³: **C 07 D 263/52,** A 01 N 43/76

(21) Anmeldenummer: 80100551.3

(22) Anmeldetag 04.02.80

(54) **Spiro-Derivate von 3-(3,5-Dihalogenphenyl)-oxazolidin-2-thion-4-onen, Verfahren zu ihrer Herstellung sowie ihre Verwendung als Fungizide.**

(30) Priorität: 15.02.79 DE 2905780

(43) Veröffentlichungstag der Anmeldung: 03.09.80 Patentblatt 80/18

(45) Bekanntmachung des Hinweises auf die Patenterteilung: 14.10.81 Patentblatt 81/41

(84) Benannte Vertragsstaaten: AT BE CH DE FR GB IT NL

(56) Entgegenhaltungen
EP-A-0 003 520
GB-A-982 940
AGRICULTURAL AND BIOLOGICAL CHEMISTRY, Band 35, Nr. 11, November 1971, Seiten 1707—1719 Tokyo, JP.
A: FUJINAMI et al.: »Studies on biological activity of cyclic imide compounds. Part I. Antimicrobial activity of 3-phenyloxazolidine-2,4-diones and related compounds«

(73) Patentinhaber: **BAYER AG, Zentralbereich Patente, Marken und Lizenzen, D-5090 Leverkusen 1, Bayerwerk (DE)**

(72) Erfinder: **Knops, Hans-Joachim, Dr., Claudiusweg 3, D-5600 Wuppertal-1 (DE)**
Erfinder: **Heine, Hans-Georg, Dr., Am Heckerhof 14, D-4150 Krefeld (DE)**
Erfinder: **Draber, Wilfried, Dr., In Den Birken 81, D-5600 Wuppertal-1 (DE)**
Erfinder: **Brandes, Wilhelm, Dr., Eichendorffstrasse 3, D-5623 Leichlingen (DE)**

**EP 0 014 887 B1**

Anmerkung Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen Er gilt erst als eingelegt, wenn die Einspruchsgebühr entrichtet worden ist (Art 99(1) Europäisches Patentübereinkommen)

BUNDESDRUCKEREI BERLIN

Spiro-Derivate von 3-(3,5-Dihalogenphenyl)-oxazolidin-2-thion-4-onen,
Verfahren zu ihrer Herstellung sowie ihre Verwendung als Fungizide

Die Erfindung betrifft Spiro-Derivate von 3-(3,5-Dihalogenphenyl)-oxazolidin-4-thion-4-onen, mehrere Verfahren zu ihrer Herstellung sowie ihre Verwendung als Fungizide.

Es ist bereits bekannt, daß Thiuramdisulfide, wie beispielsweise Tetramethyl-thiuramdisulfid, gute fungizide Eigenschaften aufweisen (vgl. US-PS 1 972 961). Die Wirkung dieser Stoffklasse ist jedoch in bestimmten Indikationsbereichen, insbesondere bei niedrigen Aufwandmengen und -konzentrationen, nicht immer voll befriedigend.

Weiterhin ist aus der Literatur schon ein Spiro-Derivat von 3-(3,5-Dihalogenphenyl)-oxazolidin-2,4-dionen bekannt [vgl. Agr. Biol. Chem. 35, 1712 (1971)]; die fungizide Wirksamkeit desselben ist jedoch nicht befriedigend.

Es wurden nun die Spiro-Derivate von 3-(3,5-Dihalogenphenyl)-oxazolidin-2-thion-4-onen der Formel I

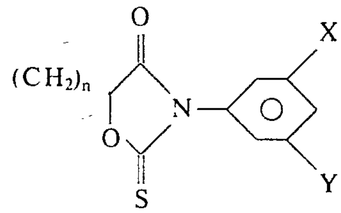

(I)

in welcher

X und Y gleich oder verschieden sind und für Halogen stehen und
n für die ganzen Zahlen 2 oder 3 steht,

gefunden.

Weiterhin wurde gefunden, daß man die Spiro-Derivate von 3-(3,5-Dihalogenphenyl)-oxazolidin-2-thion-4-onen der Formel I erhält, wenn man α-Hydroxy-cycloalkylcarbonsäuren(-ester) der Formel II

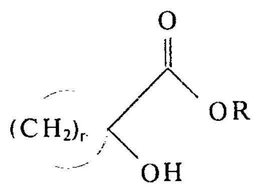

(II)

in welcher

R für Wasserstoff oder Alkyl mit 1 bis 4 Kohlenstoffatomen steht und
n die oben angegebene Bedeutung hat,

a) mit Isothiocyanaten (Senfölen) der Formel III

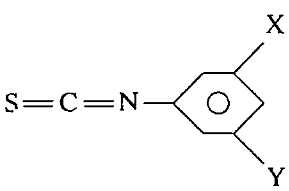

(III)

in welcher

X und Y die oben angegebene Bedeutung haben,

gegebenenfalls in Gegenwart einer Base und in Gegenwart eines Verdünnungsmittels umsetzt oder

b) mit Anilinen der Formel IV

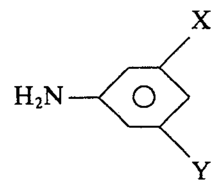

(IV)

in welcher

X und Y die oben angegebene Bedeutung haben,

in Gegenwart eines Verdünnungsmittels umsetzt und die entstehenden $\alpha$-Hydroxy-cycloalkylcarbonsäureamide der Formel V

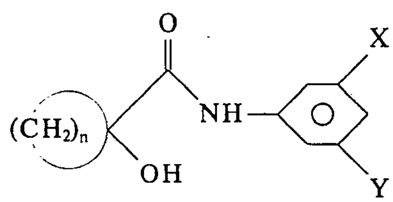

(V)

in welcher

X, Y und n die oben angegebene Bedeutung haben,

mit Thiophosgen in Gegenwart einer Base cyclisiert.

Außerdem wurde gefunden, daß die Spiro-Derivate von 3-(3,5-Dihalogenphenyl)-oxazolidin-2-thion-4-onen der Formel I gute fungizide Eigenschaften aufweisen.

Überraschenderweise zeigen die erfindungsgemäßen Spiro-Derivate von 3-(3,5-Dihalogenphenyl)-oxazolidin-2-thion-4-onen eine erheblich höhere fungizide Wirkung, insbesondere gegen Botrytis-Arten, als die aus dem Stand der Technik bekannte Verbindung Tetramethyl-thiuramdisulfid, welche ein anerkannt gutes Mittel gleicher Wirkungsrichtung ist. Die erfindungsgemäßen Wirkstoffe stellen somit eine Bereicherung der Technik dar.

Die erfindungsgemäßen Spiro-Derivate von 3-(3,5-Dihalogenphenyl)-oxazolidin-2-thion-4-onen sind durch die Formel I allgemein definiert. In dieser Formel sind X und Y gleich oder verschieden und stehen für Fluor, Chlor, Brom und Jod. Der Index n steht für die ganzen Zahlen 2 oder 3.

Bevorzugt sind insbesondere diejenigen Verbindungen der Formel I, in denen X und Y für Chlor stehen.

Im einzelnen seien außer den bei den Herstellungsbeispielen genannten Verbindungen die folgenden Verbindungen der Formel I genannt:

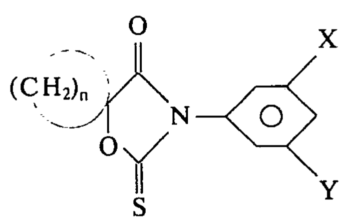

(I)

| X | Y | n |
|---|---|---|
| Cl | Cl | 3 |
| Br | Br | 2 |
| Br | Br | 3 |
| J | J | 2 |
| Cl | Br | 2 |
| Cl | Br | 3 |

Verwendet man α-Hydroxycyclopropancarbonsäure und 3,5-Dichlorphenylisothiocyanat als Ausgangsstoffe, so kann der Reaktionsablauf durch das folgende Formelschema wiedergegeben werden (Verfahren a):

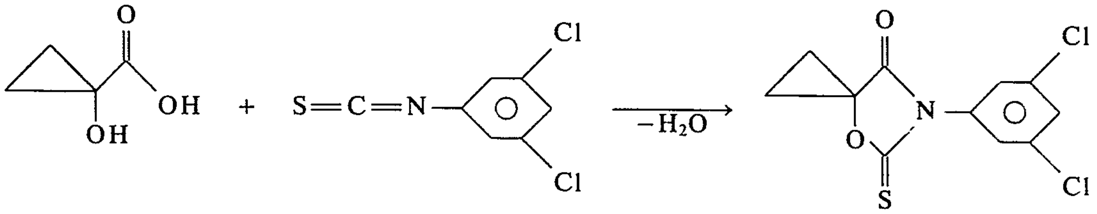


Verwendet man α-Hydroxybutancarbonsäure-ethylester und 3,5-Dibromphenylisothiocyanat als Ausgangsstoffe, so kann der Reaktionsablauf durch das folgende Formelschema wiedergegeben werden (Verfahren a):

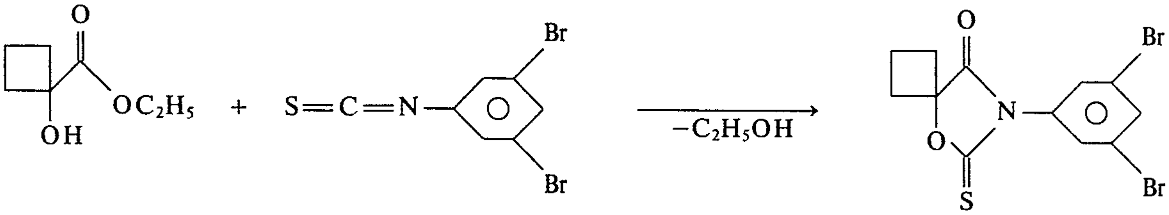


Verwendet man α-Hydroxy-cyclopropancarbonsäure, 3,5-Dichloranilin und Thiophosgen als Ausgangsstoffe, so kann der Reaktionsablauf durch das folgende Formelschema wiedergegeben werden (Verfahren b):

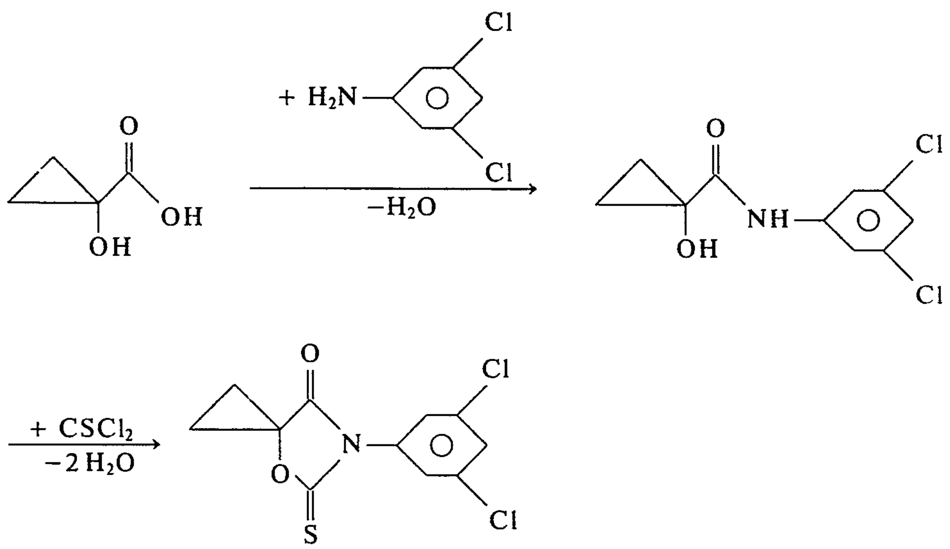


Die als Ausgangsstoffe zu verwendenden α-Hydroxy-cycloalkylcarbonsäuren(-ester) sind durch die Formel II allgemein definiert. In dieser Formel steht R vorzugsweise für geradkettiges oder verzweigtes Alkyl mit 1 bis 4 Kohlenstoffatomen, insbesondere für Methyl oder Ethyl. Der Index n steht für die ganzen Zahlen 2 oder 3.

Die Ausgangsstoffe der Formel II sind bekannt [vgl. Liebigs Ann. Chem. 1976, 463 sowie Chem. Ber. 55, 2738 (1922)]. Als Beispiele seien genannt: α-Hydroxy-cyclopropancarbonsäure, α-Hydroxy-cyclopropancarbonsäure-methyl- und ethylester, α-Hydroxy-cyclobutancarbonsäuremethyl- und ethylester und α-Hydroxy-cyclobutancarbonsäure.

Die α-Hydroxy-cyclopropancarbonsäure kann z. B. in der Weise hergestellt werden, daß man zunächst durch Acyloinkondensation von Bernsteinsäureester in Gegenwart von Trimethylchlorsilan das 1,2-Bis-(trimethylsiloxy)-1-cyclobuten darstellt, dann die erhaltene Verbindung bromiert, wobei man auf dem Wege über das 1,2-Cyclobutandion durch Ringkontraktion die 1-Hydroxy-cyclopropancarbonsäure erhält. Die 1-Hydroxy-cyclobutancarbonsäure kann z. B. dadurch hergestellt werden, daß man Cyclobutancarbonsäure bromiert und die erhaltene 1-Brom-Verbindung anschließend mit einer wäßrigen Kaliumcarbonat-Lösung behandelt.

Die weiterhin als Ausgangsstoffe zu verwendenden Isothiocyanate (Senföle) sind durch die Formel III und die Aniline durch die Formel IV allgemein definiert. In diesen Formeln sind X und Y gleich oder

verschieden und stehen für Fluor, Chlor, Brom und Jod.

Die Ausgangsstoffe der Formeln III und IV sind allgemein bekannte Verbindungen der organischen Chemie.

Für die erfindungsgemäße Umsetzung gemäß Verfahrensvariante (a) kommen als Verdünnungsmittel vorzugsweise inerte organische Lösungsmittel infrage. Hierzu gehören aromatische Kohlenwasserstoffe, wie Benzol, Toluol, Xylol oder 1,2-Dichlorbenzol, halogenierte Kohlenwasserstoffe, wie Methylenchlorid, Chloroform oder Tetrachlorkohlenstoff.

Wird die erfindungsgemäße Umsetzung gemäß Verfahrensvariante (a) in Gegenwart einer Base durchgeführt, so können alle üblicherweise verwendbaren organischen und anorganischen Basen eingesetzt werden. Hierzu gehören vorzugsweise tertiäre Amine, wie Triethylamin oder Pyridin sowie Alkoholate, wie Kalium- oder Natrium-tert.-butylat.

Die Reaktionstemperaturen können bei der erfindungsgemäßen Verfahrensvariante (a) in einem größeren Bereich variiert werden. Im allgemeinen arbeitet man zwischen 20 und 100°C, vorzugsweise bei der Siedetemperatur des jeweiligen Lösungsmittels.

Bei der Durchführung der erfindungsgemäßen Verfahrensvariante (a) arbeitet man vorzugsweise in molaren Mengen. Bei Verwendung einer Base wird diese in äquimolarer Menge eingesetzt, wenn man $\alpha$-Hydroxy-cycloalkancarbonsäuren als Ausgangsstoffe verwendet und lediglich in katalytischer Menge, wenn $\alpha$-Hydroxy-cycloalkancarbonsäureester eingesetzt werden. Zur Isolierung der Verbindungen der Formel I wird das Lösungsmittel abdestilliert und der Rückstand aufgearbeitet.

Für die erfindungsgemäße Umsetzung gemäß Verfahrensvariante (b) kommen als Verdünnungsmittel vorzugsweise inerte organische Lösungsmittel infrage. Hierzu gehören vorzugsweise die bei der Verfahrensvariante (a) bereits genannten Solventien.

Als Basen kommen für die erfindungsgemäße Umsetzung gemäß Verfahrensvariante (b) vorzugsweise die bei der Variante (a) bereits genannten Reagenzien infrage.

Die Reaktionstemperaturen können bei der erfindungsgemäßen Verfahrensvariante (b) in einem größeren Bereich variiert werden. Im allgemeinen arbeitet man zwischen 20 und 100°C, vorzugsweise bei der Siedetemperatur des verwendeten Lösungsmittels.

Bei der Durchführung der erfindungsgemäßen Verfahrensvariante (b) arbeitet man vorzugsweise in molaren Mengen. Die als Zwischenprodukte auftretenden $\alpha$-Hydroxy-cycloalkylcarbonsäureamide der Formel V können ohne Isolierung direkt umgesetzt werden. Zur Isolierung der Verbindungen der Formel I wird das Lösungsmittel und der Rückstand nach üblichen Methoden aufgearbeitet.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Botrytis-Arten, wie gegen den Erreger des Grauschimmels an Erdbeeren oder Trauben (Botrytis cinerea) verwendet werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit,

Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsform, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Beispiel A

### Botrytis-Test (Bohnen)/protektiv

Lösungsmittel: 4,7 Gewichtsteile Aceton
Dispergiermittel: 0,3 Gewichtsteile Alkyl-aryl-polyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstücke aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.

Die erhaltenen Boniturwerte werden auf Prozent Befall umgerechnet. 0% bedeutet keinen Befall, 100% bedeutet, daß der Befallsfleck vollständig ausgebildet ist.

Die Verbindung entsprechend Herstellungsbeispiel 1 zeigt hierbei eine gute, der bekannten Verbindung aus dem Stande der Technik überlegene Wirkung.

## Herstellungsbeispiele

### Beispiel 1

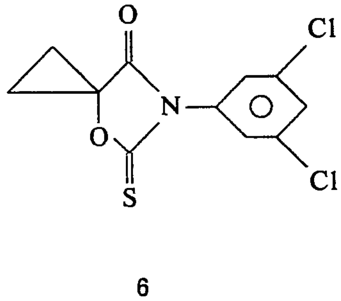

5,1 g (0,05 Mol) $\alpha$-Hydroxy-cyclopropancarbonsäure und 5,05 g Triethylamin werden in 200 ml o-Dichlorbenzol bei ca. 100° C gelöst. Man tropft eine Lösung von 10,2 g (0,05 Mol) 3,5-Dichlorphenylsenföl in 50 ml o-Dichlorbenzol hinzu und erhitzt danach 3 Stunden unter Rückfluß am Wasserabscheider. Man läßt abkühlen und engt durch Abdestillieren des Lösungsmittels im Vakuum ein. Der noch warme Rückstand wird mit 100 ml heißem Ethanol versetzt und gut durchmischt. Während des Abkühlens scheiden sich weiße Kristalle ab, die abgesaugt und aus Ethanol umkristallisiert werden.

Man erhält 6 g (45% der Theorie) 1-Oxa-3-aza-spiro[4,2]-hepta-3-(3,5-dichlorphenyl)-2-thion-4-on vom Schmelzpunkt 154° C.

**Patentansprüche**

1. Spiro-Derivate von 3-(3,5-Dihalogenphenyl)-oxazolidin-2-thion-4-onen der Formel I

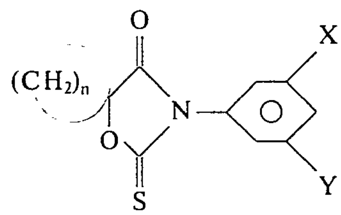

(I)

in welcher

X und Y gleich oder verschieden sind und für Halogen stehen und
n für die ganzen Zahlen 2 oder 3 steht.

2. Verfahren zur Herstellung von Spiro-Derivaten von 3-(3,5-Dihalogenphenyl)-oxazolidin-2-thion-4-onen der Formel I

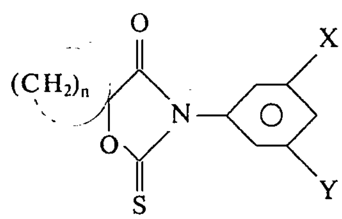

(I)

in welcher

X und Y gleich oder verschieden sind und für Halogen stehen und
n für die ganzen Zahlen 2 oder 3 steht,

dadurch gekennzeichnet, daß man $\alpha$-Hydroxy-cycloalkylcarbonsäuren(-ester) der Formel II

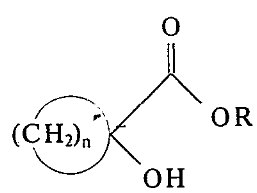

(II)

in welcher

R für Wasserstoff oder Alkyl mit 1 bis 4 Kohlenstoffatomen steht und
n die oben angegebene Bedeutung hat,

a) mit Isothiocyanaten (Senfölen) der Formel III

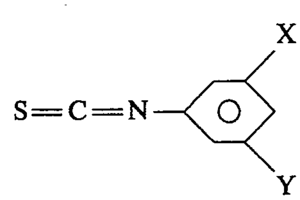

(III)

in welcher

X und Y die oben angegebene Bedeutung haben,

gegebenenfalls in Gegenwart einer Base und in Gegenwart eines Verdünnungsmittels umsetzt oder

b) mit Anilinen der Formel IV

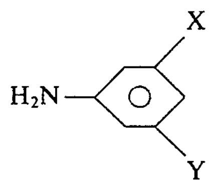

(IV)

in welcher

X und Y die oben angegebene Bedeutung haben,

in Gegenwart eines Verdünnungsmittels umsetzt und die entstehenden α-Hydroxy-cycloalkylcarbonsäureamide der Formel V

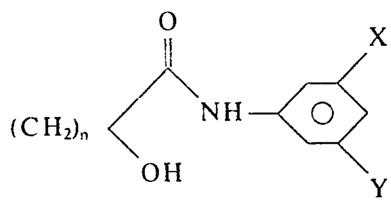

(V)

in welcher

X, Y und n die oben angegebene Bedeutung haben,

mit Thiophosgen in Gegenwart einer Base cyclisiert.

3. Fungizide Mittel, gekennzeichnet durch einen Gehalt an mindestens einem Spiro-Derivat von 3-(3,5-Dihalogenphenyl)-oxazolidin-2-thion-4-onen der Formel I in Ansprüchen 1 und 2.

4. Verwendung von Spiro-Derivaten von 3-(3,5-Dihalogenphenyl)-oxazolidin-2-thion-4-onen der Formel I in Ansprüchen 1 und 2 zur Bekämpfung von Pilzen.

5. Verfahren zur Herstellung von fungiziden Mitteln, dadurch gekennzeichnet, daß man Spiro-Derivate von 3-(3,5-Dihalogenphenyl)-oxazolidin-2-thion-4-onen der Formel I in Ansprüchen 1 und 2 mit Streckmitteln und/oder oberflächenaktiven Mitteln vermischt.

**Claims**

1. Spiro derivatives of 3-(3,5-dihalogenophenyl)-oxazolidine-2-thion-4-ones of the formula

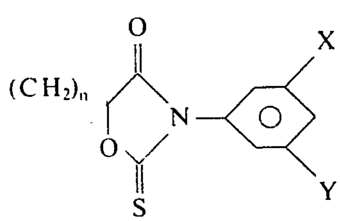

(I)

in which

X and Y are identical or different and represent halogen and
n represents the integers 2 or 3.

2. Process for the preparation of spiro derivatives of 3-(3,5-dihalogenophenyl)-oxazolidine-2-thion-4-ones of the formula

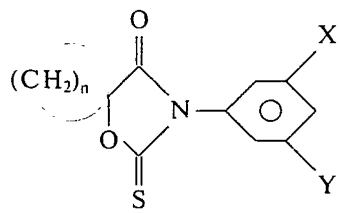

(I)

in which

X and Y are identical or different and represent halogen and
n represents the integers 2 or 3,

characterised in that α-hydroxy-cycloalkylcarboxylic acids or acid esters of the formula II

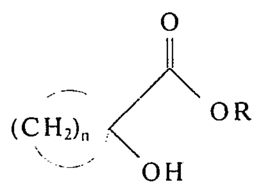

(II)

in which

R represents hydrogen or alkyl with 1 to 4 carbon atoms and
n has the meaning indicated above,

a) are reacted with isothiocyanates of the formula III

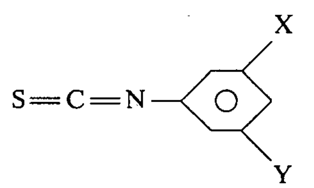

(III)

in which

X and Y have the meaning indicated above,

if appropriate in the presence of a base and in the presence of a diluent, or

b) are reacted with anilines of the formula IV

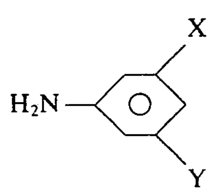

(IV)

in which

X and Y have the meaning indicated above,

in the presence of a diluent, and the α-hydroxy-cycloalkylcarboxylic acid amides formed, of the formula

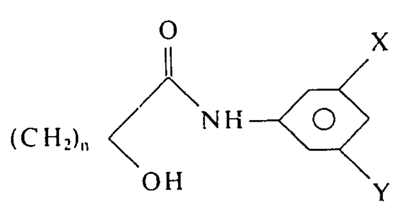

(V)

in which

X, Y and n have the meaning indicated above,

are cyclised with thiophosgene in the presence of a base.

3. Fungicidal agents, characterised in that they contain at least one spiro derivative of 3-(3,5-dihalogenophenyl)-oxazolidine-2-thion-4-ones of the formula I in Claims 1 and 2.

4. Use of spiro derivatives of 3-(3,5-dihalogenophenyl)-oxazolidine-2-thion-4-ones of the formula I in Claims 1 and 2 for combating fungi.

5. Process for the preparation of fungicidal agents, characterised in that spiro derivatives of 3-(3,5-dihalogenophenyl)-oxazolidine-2-thion-4-ones of the formula I in Claims 1 and 2 are mixed with extenders and/or surfaceactive agents.

**Revendications**

1. Spiro-dérivés de 3-(3,5-dihalogénophényl)-oxazolidine-2-thione-4-ones de formule:

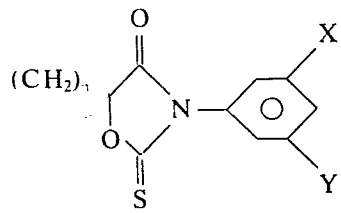

(I)

dans laquelle

X et Y sont identiques ou différents et représentent de l'halogène et
n représente les nombres entiers 2 ou 3.

2. Procédé de fabrication de spiro-dérivés de 3-(3,5-dihalogénophényl)-oxazolidine-2-thione-4-ones de formule:

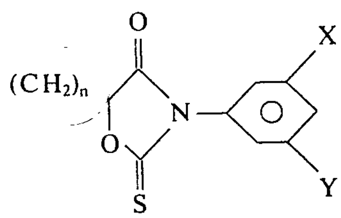

(I)

dans laquelle

X et Y sont identiques ou différents et représentent de l'halogène et
n représente les nombres entiers 2 ou 3,

caractérisé en ce qu'on fait réagir des acides (esters) $\alpha$-hydroxy-cycloalcoyl-carboxyliques de formule II:

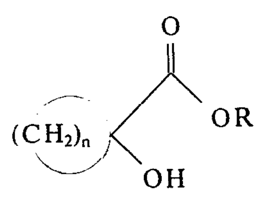

(II)

dans laquelle

R représente de l'hydrogène ou un alcoyle ayant 1 à 4 atomes de carbone et
n a la signification indiquée plus haut,

a) avec des isothiocyanates (huiles moutarde) de formule III

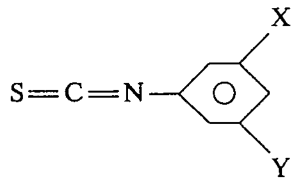

(III)

dans laquelle

X et Y ont la signification indiquée plus haut,

éventuellement en présence d'une base et en présence d'un diluant, ou

b) avec des anilines de formule IV

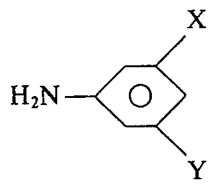

(IV)

dans laquelle

X et Y ont la signification indiquée plus haut,

en présence d'un diluant et en ce qu'on cyclise les $\alpha$-hydroxy-cycloalcoyl-carboxamides de formule:

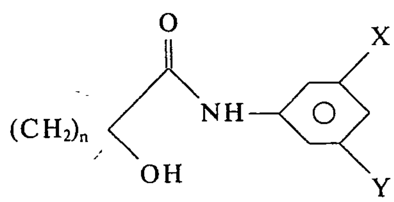

(V)

dans laquelle

X, Y et n ont la singification indiquée plus haut,

avec du thiophosgène en présence d'une base.

3. Agents fongicides, caractérisés par une teneur en au moins un spirodérivé de 3-(3,5-dihalogénophényl)-oxazolidine-2-thione-4-ones de formule I selon les revendications 1 et 2.

4. Utilisation de spiro-dérivés de 3-(3,5-dihalogénophényl)-oxazolidine-2-thione-4-ones de formule I selon les revendications 1 et 2 pour combattre les champignons.

5. Procédé de fabrication d'agents fongicides, caractérisé en ce qu'on mélange des spirodérivés de 3-(3,5-dihalogénophényl)-oxazolidine-2-thione-4-ones de formule I selon les revendications 1 et 2 avec des diluants et/ou des agents tensioactifs.